# EUROPEAN PATENT APPLICATION

(11) **EP 2 444 479 A1**
(43) Date of publication of application: **25.04.2012**
(21) Application number: 10789186.3
(22) Date of filing: 09.06.2010
(51) Int. Cl.: C12M 1/34, G01N 21/17, G01N 21/64, G06F 17/30, G06Q 50/00, G06T 1/00

(54) **STATE DETERMINATION METHOD FOR CELL CLUSTER, IMAGE PROCESSING PROGRAM AND IMAGING PROCESSING DEVICE USING SAID METHOD, AND METHOD FOR PRODUCING CELL CLUSTER**

(30) Priority: 19.06.2009 JP 2009146149
(71) Applicant: Nikon Corporation, Tokyo 100-8331 (JP)
(72) Inventor: MIMURA, Masafumi, Tokyo 100-8331 (JP); YANO, Kazuhiro, Tokyo 100-8331 (JP); ITO, Kei, Tokyo 100-8331 (JP); SASAKI, Hideki, Tokyo 100-8331 (JP)
(74) Representative: HOFFMANN EITLE
(86) International application number: PCT/JP2010/003832
(87) International publication number: WO 2010/146802

(57) **Abstract**

Provided is means by which the state of change of a cell aggregation toward becoming multi-layered can be decided from two observation images taken at a predetermined time interval. An image processing program (GP) is provided with a step (S10) for obtaining a first image and a second image taken by an imaging device at a predetermined time interval; a step (S40) for performing block matching, using the luminance distribution of a local region of the first image as a standard, in a vicinity including the corresponding position of the second image, taking the degree of approximation of the region with the highest degree of matching as a representative degree of approximation, sequentially moving the local region, and calculating the representative degree of approximation for individual parts of the cell aggregation; and a step (S50) for outputting multi-layering information corresponding to the calculated representative degree of approximation. The image processing program (GP) is configured so as to output multi-layering information by which the state of change of the cell aggregation toward becoming multi-layered can be decided from the first and second images.

## Description

### TECHNICAL FIELD

The present invention relates to a state analysis technique for determining the state of change of a cell aggregation toward becoming multi-layered, from time-lapse images obtained during cell observation.

### TECHNICAL BACKGROUND

A cell culture microscopy can be cited as an example of a device for observing a cell while the cell is being cultured. A cell culture microscopy is provided with a cell culture device for forming an environment suitable for culturing a cell, and a microscope observation system for microscopic observation of a cell in a cell culture container. The cell culture microscopy is configured so that changes, divisions, and other cell activities can be observed while the living cell is cultured (see Patent Document 1, for example). During the process of culturing a live cell, a cell aggregation is formed by the progression of cell division. During the initial process of cell division, the divided cells spread out in the horizontal direction throughout the cell culture medium in a single-layered state, but as the activity of cell division intensifies and the cell aggregation matures, the cells also spread out in the up-down direction so as to form bubbles, and the ''multi-layering'' progresses.

In a conventional cell observation technique using a cell culture microscopy, the state of change of a cell aggregation toward becoming multi-layered is judged by a visual judgment, in which a determination is made by visual observation of a microscope observation image, and/or by a reagent judgment, in which, a reagent is administered and a determination is made from the state of coloration or other parameter.

### PRIOR ARTS LIST

### PATENT DOCUMENTS

Patent Document 1: Japanese Laid-open Patent Publication No. 2004-229619 (A)

### SUMMARY OF THE INVENTION

### PROBLEMS TO BE SOLVED BY THE INVENTION

However, in order to extract a cell aggregation and determine, from a plurality of time lapse images, the state in which the cell aggregation has became multi-layered, the technique for visual judgement that has conventionally been employed requires that an expert having a certain amount of experience make the determination over a period of time. In particular, in a case where the observation image at each instance includes a plurality of cell aggregations, determining the state (s) of change toward becoming multi-layered while still identifying individual cell aggregations is a very complex task. The visual judgment is also problematic in that it is difficult to quantitatively ascertain the position and/or size of a site in a cell aggregation that has become multi-layered (the ratio of the surface area and/or cell aggregations, or another parameter) . The technique for a reagent judgment has been further problematic in that administering the reagent has a major chemical and physical effect on the cells, and in that there are major constraints in using cultured cells.

The present invention was developed in view of such problems, it being an object of the present invention to provide means by which, the state of change of a cell aggregation toward becoming multi-layered can be determined, from a small number of images taken by an imaging device without there being damage to the cells due to the administration of a reagent.

### MEANS TO SOLVE THE PROBLEMS

According to a first aspect illustrating an example of the present invention, there is provided a technique for determining the state of a cell aggregation comprising the steps of obtaining a first image and a second image taken by photographing a cell aggregation at a predetermined time interval by an imaging device; performing block matching, using the luminance distribution of a local region in the cell aggregation of the first image as a standard, of the luminance distribution in a nearby part including a corresponding position in the cell aggregation of the second image, the degree of approximation of the region exhibiting the highest degree of matching serving as a representative degree of approximation of the region of the relevant position; moving the local region in the first image and calculating the representative degree of approximation of individual parts of the cell aggregation; and deciding a state of change of the cell aggregation toward becoming multi-layered in accordance with the calculated representative degree of approximation of each of the parts of the cell aggregation.

According to a second aspect illustrating an example of the present invention, there is provided an image processing program that can be read out by a computer, the image processing program being adapted for causing the computer to function as an image processing device for obtaining an image taken by an imaging device and performing image processing, comprising the steps of obtaining a first image and a second image taken by photographing a cell aggregation at a predetermined time interval by an imaging device; performing block matching, using the luminance distribution of a local region in the cell aggregation of the first image as a standard, of the luminance distribution in a vicinity including a corresponding position in the cell aggregation of the second image, having the degree of approximation of the region exhibiting the highest degree of matching serve as a representative degree of approximation of the region of the relevant position, moving the local region in the first image, and calculating the representative degree of approximation of individual parts of the cell aggregation; and outputting multi-layering information in accordance with the calculated representative degree of approximation; the image processing program being adapted for causing the computer to function so as to output the multi-layering information by which the state of change of a cell aggregation toward becoming multi-layered can be determined from the obtained first image and the second image.

According to a third aspect illustrating an example of the present invention, there is configured an image processing device provided with an image analysis unit for obtaining a first image and a second image taken by photographing a cell aggregation at a predetermined time interval by an imaging device, and analyzing the images, and an output unit for outputting the analysis results from the image analysis unit, the image processing device being configured such that the image analysis unit performs block matching, using the luminance distribution of a local region in the cell aggregation of the first image as a standard, of the luminance distribution in a vicinity including a corresponding position in the cell aggregation of the second image, has the degree of approximation of the region exhibiting the highest degree of matching serve as a representative degree of approximation of the region of the relevant position, movers the local region in the first image, calculates the representative degree of approximation of individual parts of the cell aggregation, and generates multi-layering information in accordance with the calculated representative degree of approximation of each of the parts of the cell aggregation; and the output unit outputs multi-layering information of the cell aggregation generated by the image analysis unit.

In the present invention described above, the degree of approximation is preferably a correlation value, and, in a case where the correlation value of the representative degree of approximation is at or below a threshold, multi-layering is decided to have occurred in the relevant site. The degree of approximation is also preferably a difference, and in a case where the difference of the representative degree of approximation is at or above a threshold, multi-layering is decided to have occurred in the relevant site.

The image processing program or image processing device of the present invention, in a preferred configuration, outputs position information for a cell aggregation at a site where multi-layering is decided to have occurred, and, in a preferred configuration, outputs information on the size of the cell aggregation that accounts for the site where multi-layering is decided to have occurred (the surface area, the volume, the ratio thereof, or the like). In a preferred configuration, in a case where the image includes a plurality of cell aggregations, the state of a change toward becoming multi-layered is determined for each cell aggregation; a distinction is made between cell aggregations having multi-layered sites and cell aggregations not having multi-layered sites, and the results of the distinction are outputted.

According to a fourth aspect illustrating an example of the present invention, there is provided a method for producing a cell aggregation, comprising a cell culture step for culturing cells, and a determination step for observing, by using the image processing device described above, the cells cultured in the cell culture step, and determining the state of change of a cell aggregation toward becoming multi-layered in the cells, which vary by cell culture.

According to a fifth aspect illustrating an example of the present invention, there is provided a method for producing a cell aggregation, comprising a cell culture step for culturing cells; an obtainment step for obtaining a first image and a second image taken by photographing, at a predetermined time interval by an imaging device, the cells cultured in the cell culture step; a degree of approximation setting step for performing block matching, using the luminance distribution of a local region in the cell aggregation of the first image as a standard, of the luminance distribution in a nearby part including a position corresponding to the local region in the cell aggregation of the second image, and having the degree of approximation of the region exhibiting the highest degree of matching serve as a representative degree of approximation of the relevant local region; a calculation step for moving the local region in the first image, and calculating the representative degree of approximation of individual parts of the cell aggregation; and a determination step for determining the state of change of a cell aggregation toward becoming multi-layered in accordance with the representative degree of approximation of each of the parts of the cell aggregation as calculated in the calculation step.

### ADVANTAGEOUS EFFECTS OF THE INVENTION

In the technique for determining the state of a cell aggregation, the image processing program, and the image processing devise of the present invention, a first image and a second image in which images of a cell aggregation are taken at a predetermined time interval by an imaging device are subjected to block matching, in which the luminance distribution of a local region of the first image is used as a standard, and the state of change of a cell aggregation toward becoming multi-layered is determined on the basis of a calculated representative degree of approximation of each part of the cell aggregation. Therefore, according to the present invention, there can be provided means by which the state of change of a cell aggregation toward becoming multi-layered can be determined from a small number of images taken by an imaging device without the cells being damaged due to the administration of a reagent.

### BRIEF DESCRIPTION OF THE DRAWINGS

FIG. 1 is a flow chart illustrating an example of an overview of the configuration of an image processing program;
FIG. 2 is a diagram providing a rough structural view of a cell culture observation system illustrated as an example of the application of the present invention;
FIG. 3 is a block diagram of the aforementioned cell culture observation system;
FIG. 4 is a block diagram illustrating an overview of an example of a configuration of an image processing device;
FIG. 5A is a first image and FIG. 5B is a second image of a cell aggregation, the first image and the second image taken at a predetermined time interval;
FIG. 6 is a schematic illustrating an example of the status of a cell aggregation that has been segmented and labeled;
FIG. 7A is an example of a configuration of a local region that is set in the first image, and FIG. 7B is an explanatory view to depict the status in which block matching is executed for a nearby part that includes a corresponding position in the second image;
FIG. 8A is an explanatory view illustrating an example of the size of a local region relative to the cell aggregation, and FIG. 8B is an example of a configuration for displaying the multi-layering information calculated by the image analysis; and
FIG. 9 is a flow chart illustrating a method for producing a cell aggregation.

### DESCRIPTION OF THE EMBODIMENTS

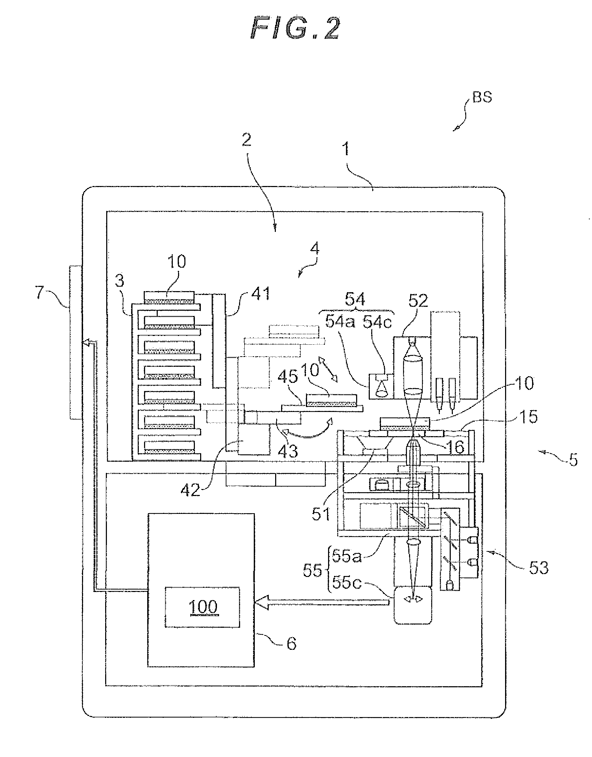

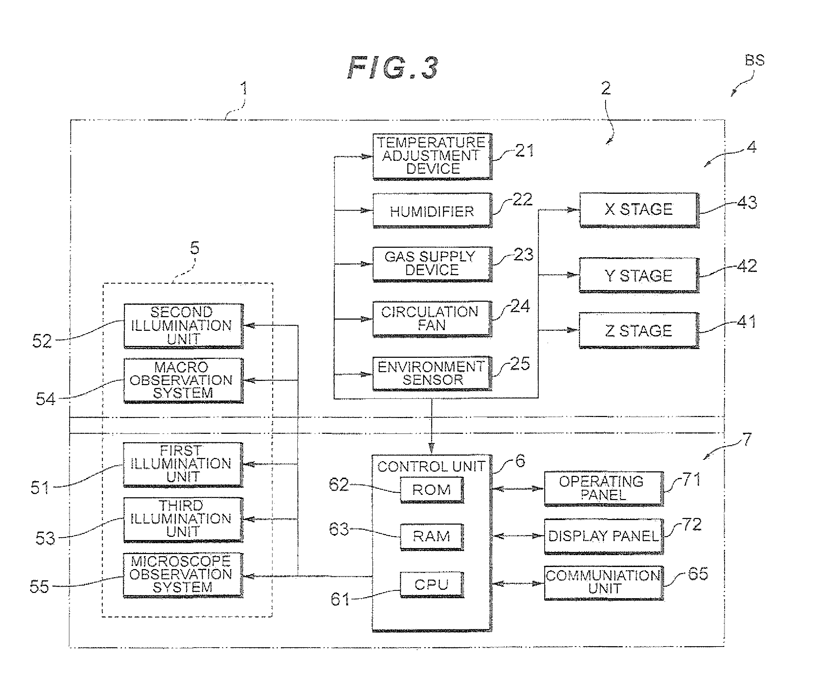

Embodiments of the present invention will be described hereinafter with reference to the accompanying drawings. As an example of a system in which the image processing device of the present invention has been applied, FIGS. 2 and 3 illustrate a rough structural view and a block diagram of a cell culture observation system. First, a description of the overall configuration of a cell culture observation system BS will be summarized.

The cell culture observation system BS is broadly constituted of a cell culture chamber 2 provided to a top part of a chassis 1; a stocker 3 for accommodating and retaining a plurality of cell culture containers 10; an observation unit 5 for observing samples in the cell culture containers 10; a conveyance unit 4 for conveying the cell culture containers 10; a control unit 6 for controlling the operation of the system; an operating board 7 provided with an image display device; and other components.

The cell culture chamber 2 is a compartment for forming a cell culture environment, and the cell culture chamber 2 is additionally provided with such components as a temperature adjustment device 21; a humidifier 22; a gas supply device 23 for supplying CO2 gas, N2 gas, or other gas; a circulation fan 24; and an environment sensor 25 for detecting the temperature, humidity, and other features of the cell culture chamber 2. The stocker 3 is formed in a shelf shape partitioned in the front-rear and up-down directions, a specific number being set for each shelf. The cell culture container 10 is appropriately selected according to the type or purpose of the cell to be cultured; cell samples are injected together with a liquid cell culture medium and retained in, for example, dish-type cell culture containers. A code number is assigned to each of the cell culture containers 10, which are associated with a designated number and accommodated in the stocker 3. The conveyance unit 4 comprises such components as a Z stage 41 capable of moving up and down, a Y stage 42 capable of moving forward and backward, and an X stage 43 capable of moving left and right, these stages being provided within the cell culture chamber 2. A support arm 45 for lifting and supporting a cell culture container 10 is provided toward the distal end of the X stage 43.

The observation unit 5 is constituted of such components as a first illumination unit 51 for illuminating a sample from a lower side of a sample stage 15; a second illumination unit 52 for illuminating the sample along the optical axis of a microscope observation system 55 from above the sample stage 15; a third illumination unit 53 for illuminating the sample from below; a macro observation system 54 for macro observation of the sample; a microscope observation system 55 for micro observation of the sample; and an image processing device 100. A transparent window part 16 is provided to the sample stage 15, in the region thereof observed by the microscope observation system 55.

The macro observation system 54 is configured to have an observation optical system 54a and a CCD camera or other imaging device 54c for taking an image of a sample that is imaged by the observation optical system. An overall observation image (macro image) is obtained from above the cell culture container 10, which is backlit by the first illumination unit 51. The microscope observation system 55 is configured to have an observation optical system 55a comprising an objective lens, a middle zooming lens, a fluorescence filter, and other components; and a cooled CCD camera or other imaging device 55c for taking an image of the sample imaged by the observation optical system 55a. The objective lenses and middle zooming lenses are provided in pluralities, and are configured such that the desired magnification for observation can be set by altering the combination of lenses. The microscope observation system 55 obtains a transmittance image of a cell illuminated by the second illumination unit 52; a reflection image of a cell illuminated by the third illumination unit 53; a fluorescence image of a cell illuminated by the third illumination unit 53, and other microscope observation images (micro images) in which the cell inside the cell culture container 10 is microscopically observed.

Images are taken by the imaging device 54c of the macro observation system 54 and the imaging device 55c of the microscope observation system 55, the image processing device 100 processing the signals inputted from these imaging devices; and generating an image of the overall observation image, the microscope observation image, or the like. The image processing device 100 applies image analysis to the (image data of the) observation images, generates a time lapse image, predicts a movement direction of a cell, analyzes the degree of activity of a cell, analyzes the motion state of the cell, analyzes the state of change of a cell aggregation toward becoming multi-layered, and performs other processing. The image processing device 100 will be described in detail hereinafter.

The control unit 6 has a CPU 61 for executing processes; a RCM 62 in which a control program, control data, or the like for the cell culture observation system BS are set and stored; and a RAM 63 for temporarily storing observation conditions, image data, and the like, which comprises a hard drive, DVD, or other auxiliary storage device; and other components; and controls operation of the cell culture observation system BS. Therefore, as illustrated in FIG. 3, the respective constituent instruments of the cell culture chamber 2, the conveyance unit 4, the observation unit 5, and the operating board 7 are connected to the control unit 6. Environment conditions of the cell culture chamber 2, an observation schedule, and observation classifications, observation positions, observation magnifications, and other information for the observation unit 5 are set and stored in the RAM 63, in accordance with the observation program. The RAM 63 is also provided with an image data memory region for recording image data taken by the observation unit 5. Index data, which include a code number of the cell culture container 10, an image-capture date and time, and other information, are recorded in association with image data.

The operating board 7 is provided with an operating panel 71 to which a keyboard, switch, or other input/output instrument is provided; and with a display panel 72 for displaying an operating screen, an observation image, analysis results, or the like. On the operating panel 71, the observation program is set, the conditions are selected, and an operational instruction or the like is inputted. A communication unit 65 is configured to conform to a wired or wireless communication standard, permitting data to be sent from and received by a computer or the like that is externally connected to the communication unit 65.

In the cell culture observation system BS thus generally configured, the CPU 61 controls the operation of each of the components and automatically photographs the sample in the cell culture container 10, in accordance with the observation program that has been set in the operating board 7. When the observation program is started, the CPU 61 controls the operation of the temperature adjustment device 21, the humidifier 22, and the like, on the basis of the environment conditions stored in the RAM 63. The observation conditions stored in the RAM 63 are read in; the X, Y, and Z stages 43, 42, 41 are operated on the basis of the observation schedule; the cell culture container 10 that is to be observed is conveyed from the stocker 3 to the sample stage 15; and the observation by the observation unit 5 is initiated. In a case where, for example, the observation that has been set in the observation program is micro observation of a cell, the corresponding cell culture container 10 is positioned onto the optical axis of the microscopic observation system 55, the light source of the second, illumination unit 52 or the third illumination unit 53 is activated, and the imaging device 55c is made to take a microscopic observation image.

The cell culture observation system BS configured as described above has a function whereby the image processing device 100 obtains a plurality of images by an image processing device (54c, 55c) , the plurality of images being taken at a predetermined time interval, and determines the state of change toward becoming multi-layered of a cell aggregation included in the image. The cell culture observation system BS is used appropriately to analyze, for example, iPS cells, ES cells, or the like.

In the image processing device 100, two observation images of a cell aggregation taken at a predetermined time interval are used, and block matching is performed, where the luminance distribution of a partial region (local region) of the cell aggregation at a previous time serves as the standard, relative to a peripheral part that includes the position thereof at a subsequent time, and an evaluation of the state of change toward becoming multi-layered is decided using the degree of approximation of the region with the greatest degree of matching (the region of the position having the least change in the luminance distribution within the region) as the representative degree of approximation of the region of the relevant position. Such a technique makes use of the fact that the image at a site where the cells have not become multi-layered (a single-layer region) and the image at a site where the cells have become multi-layered have the following characteristics.

Although a cell aggregation is a plurality of cells that have aggregated, in a single-layered cell aggregation wherein cells aggregate in a simple manner and spread out in the horizontal direction, the size of the cells and the boundaries between the cells can be observed even when the original individual cells are in some degree of a mobile and/or rotary configuration; and the structure thereof is presumably retained. On the other hand, in a case where the cells become multa-layered, changes occur such that bubbles form through division and/or movement in the up-down direction in the interior of the cell aggregation; therefore, the spatial structure and brightness of the images change dramatically.

Thus, in a single-layered region, the changes in the interior of the cell aggregation are primarily the spatial movement; therefore, performing block matching in the periphery of a corresponding position of two images achieves a higher degree of matching. By contrast, in a multi-layered region, the changes in the interior of the cell aggregation involve not only spatial movement but also structural changes, and therefore result in a lower degree of matching even when the periphery is searched. For example, in a case where a correlation value is used as the degree of approximation, a single-layered region has a high representative degree of approximation. By contrast, a multi-layered region, in which the cells change so as to form bubbles, has a lower representative degree of approximation, and the state of change toward becoming multi-layered can be decided depending on the size of the representative value of approximation.

In the present invention, attention being drawn to such characteristics of a single-layered region and a multi-layered region when imaged, image processing is performed on the two observation images of a cell aggregation separated by a predetermined time interval whereby the state of change toward becoming multi-layered is determined. The image processing device 100 uses the luminance distribution of a local region in a cell aggregation of the observation image at a previous time (which, in this description, is the "first image") as the standard to perform block matching of the luminance distribution in a nearby part, which includes the corresponding position in the cell aggregation, of the observation image at a subsequent time (which, similarly, is the "second images"). Taking the degree of approximation of the region with the greatest degree of matching as the representative degree of approximation of the region of the relevant position, the local region in the first image is sequentially moved within the image, the representative degree of approximation of each part of the cell aggregation is calculated, and same is output such that the state of change of the cell aggregation toward becoming multi-layered can be decided on the basis of the calculated representative degree of approximation of each part of the cell aggregation.

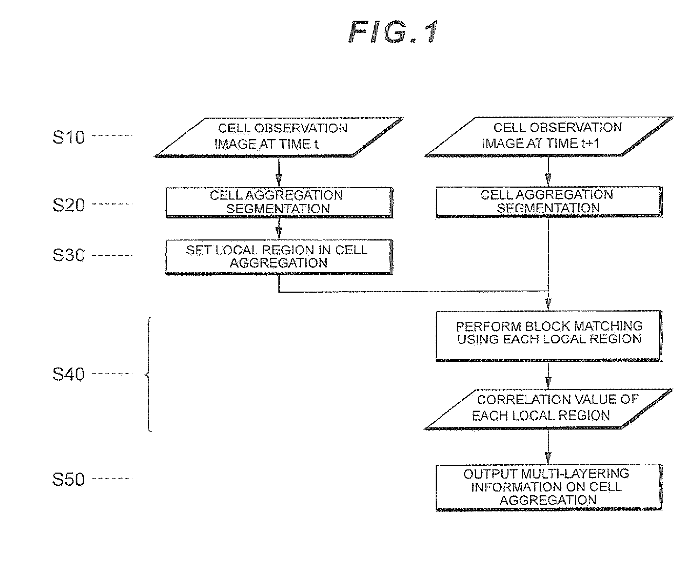

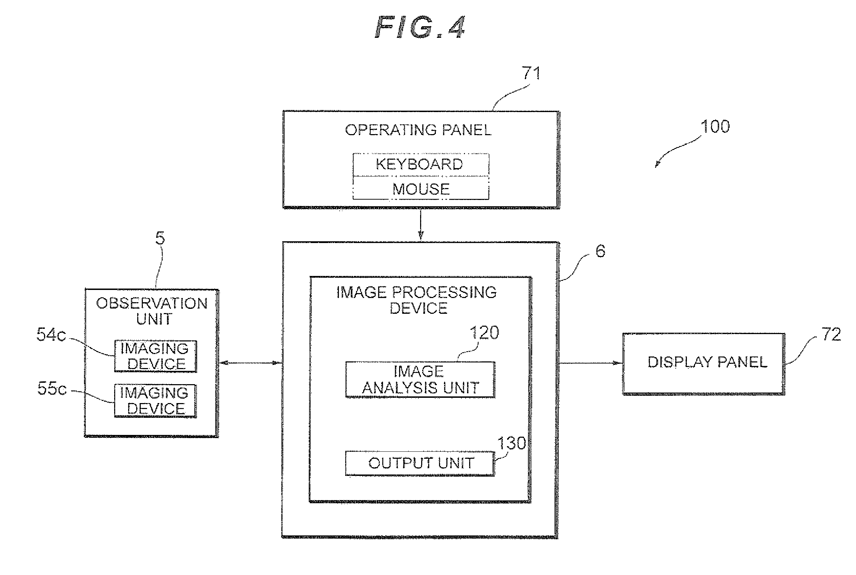

FIG. 4 illustrates a block view of the image processing device 100, and FIG. 1 illustrates a flow chart of the image processing program GP for processing the determination of the state of change toward becoming multi-layered as described above.

The image processing device 100 is configured to be provided with an image analysis unit 120 for obtaining an image of a cell aggregation taken by an imaging device (55c, 54c) and analyzing the images, and an output unit 130 for outputting the analysis results from the image analysis unit. The image processing device 100 is configured such that the analysis results from the image analysis unit 120 are outputted from the output unit 130 and displayed on the display panel 72 or the like; for example, information on the position and/or size of a site where multi-layering is estimated to have occurred (surface area, volume, the ratio thereof, or another parameter), the estimated degree of multi-layering, a determination between a cell aggregation that includes and a cell aggregation that does not include a multi-layered site, or the like.

The image processing program GP, which is set and stored in the ROM 62, is read into the CPU 61, and processing based on the image processing program GP is executed sequentially by the CPU 61, whereby the image processing device 100 is configured. In other words, the image processing program GP is software serving to cause the CPU 61 (a computer), which is a hardware resource, to function as the image processing device 100.

The image analysis unit 120 runs the following image processing on the basis of the image processing program GP for the images of the cell aggregation, which are taken by an imaging device for the purpose of description, refers here to the imaging device 55c of the micro system and recorded in the RAM 63. When the second image has been taken by the imaging device 55c, the state of change of the cell aggregation toward becoming multi-layered at the current point in time may also be subjected to image processing and outputted in real time, from the first image, which is recorded in the RAM 63, and the second image, which has been obtained anew.

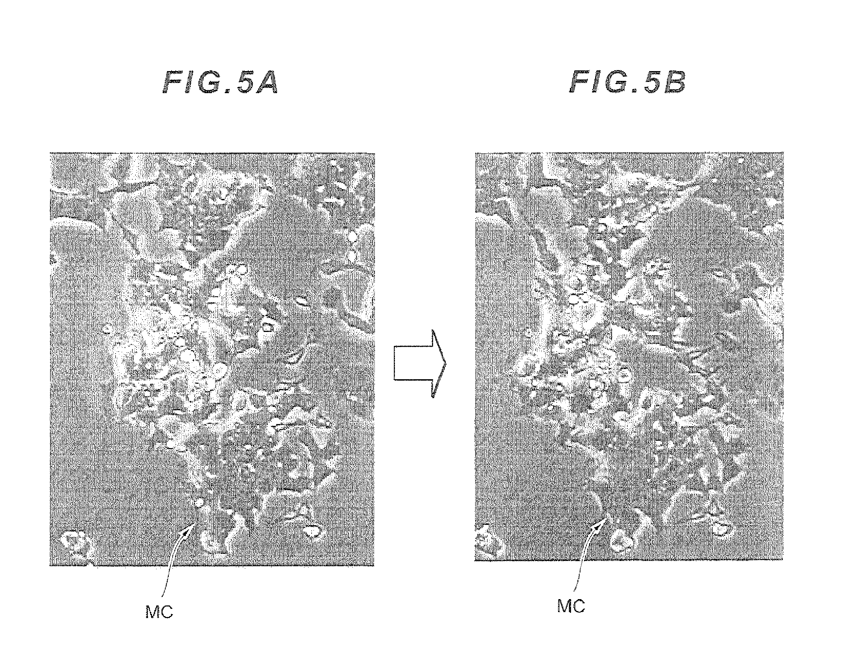

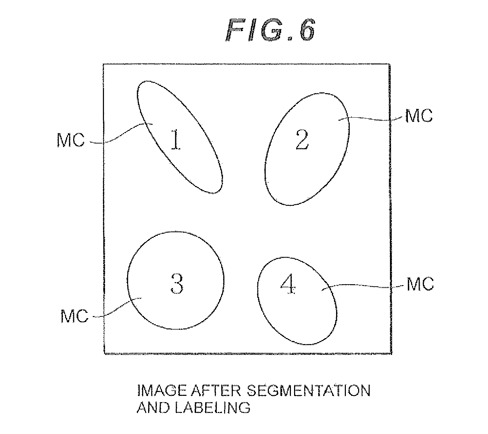

The image analysis unit 120 obtains, in step S10, a cell observation image at a time t that is stored in the RAM 63 (the first image illustrated in FIG. 5A) and a cell observation image at a subsequent time t+1 at a predetermined time interval (the second image illustrated in FIG. 5B), and, in step S20, segments the cell aggregations MC by the level set method and variance filtering for each of the images. At such a time, as illustrated in FIG. 6, in a case where the image includes a plurality of segmented cell aggregations MC, each of the cell aggregations MC are labeled and associations are made for the cell aggregations between the first Image and the second image. For example, the cell aggregations MC given the labels 1, 2, 3 ... in each of the images are associated, where a label that overlaps between images represents the same cell aggregation. The aforementioned predetermined time interval is appropriately selected in accordance with the type and/or activity status of the cells that are to be observed, but the images are selected over an interval of time on the order of ten minutes to one hour in a case where the cells are very active, or on the order of 30 minutes to two hours in a case where the cells are not very active.

Next, the cell aggregations MC are aligned in order to reduce the effects of cases in which the cell aggregations move from the first image to the second image (not shown). The position of the center of gravity of the cell aggregation, the vertex positions of the rectangular contour thereof, or the like can be used as a standard for alignment; the angle of rotation of the cell aggregations can be accounted for so as to maximize the correlation of the moment of the shape (minimize the difference), whereby the effects of rotation can be reduced. The alignment may be done at the position and angle at which the difference of the contour shapes and/or luminance distribution of each of the cell aggregations reaches a minimum (the correlation value reaches a maximum).

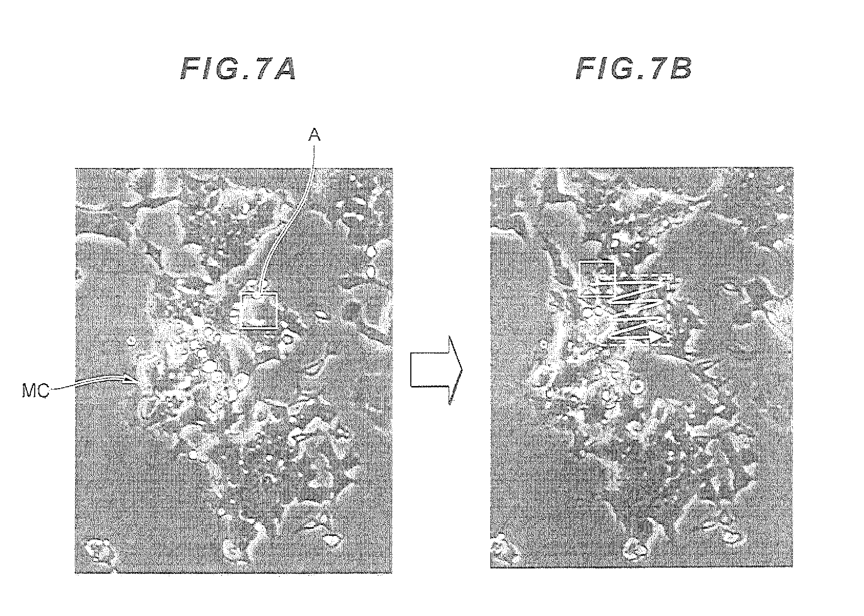

In step S30, a local region A centered on the pixels forming the first image is set in the cell aggregation MC of the image. The "local region" A, which in FIG. 7A is illustrated enclosed by a white-bordered box, is set sufficiently smaller than the size of the cell aggregation, and is set to, for example, approximately 5 × 5 to 15 × 15 pixels (which is the approximate size of several cells). The setting of the position of the local region can be configured so as to be an automatic setting where the contour edges of the cell aggregations that have been segmented in step S20 serve as starting point; in addition, in a case where, for example, an operator uses a mouse or the like to designate an analysis range and executes analysis for a specific portion of a cell aggregation (a portion of interest), the configuration may be such that the edges or middle of the set analysis range are set as starting points.

Block matching is performed in step S40 for the local region set in this manner. The block matching uses the luminance distribution of the local region (block) A set in the first image (see FIG. 7A) as a standard, scanning the luminance distribution of the local region A relative to the periphery that includes the region of the corresponding position in the second image, as illustrated in FIG. 7B, and calculating the degree of approximation at each of the positions to search for the position that has the highest degree of matching.

A correlation value, difference, multiplication value, or other value of the luminance distribution can be used as a criterion to evaluate the degree of approximation; for example, a case in which the correlation value is used involves a search for the position having the greatest correlation value (approaching 1), and a case in which the difference is used as the evaluation criterion involves a search for the position having the smallest difference (approaching 0). Then, the degree of approximation of the position having the greatest degree of matching is recorded in the RAM 63 as the representative degree of approximation of the relevant position. There follows a description of a case in which the correlation value is used as the degree of approximation.

In a case where the local region has a single-layered structure, the changes of a cell aggregation over time are primarily composed of cellular movement; therefor, performing block matching at a peripheral part that includes the corresponding position results in a high value for the correlation value of the representative degree of correlation. On the other hand, in a case where the local region is a site that has become multi-layered, the changes of the cell aggregation over time involve deformation of the spatial structure and luminance shifts; therefore, the correlation value of the representative degree of approximation is small even when the periphery is searched.

In step S40, the local region of the first image, serving as a comparative standard, is moved a predetermined number of pixels (a single pixel or a plurality of pixels) within the image to perform sequential block matching, and the representative degree of approximation of each of the parts is calculated for the entire region of the cell aggregation (the entire region of the observation range in a case where an analysis range is designated). In step S50, the representative degrees of approximation of each of the parts of the cell aggregation obtained in step S40 are converted to multi-layering information by which a decision can be made as regards the state of change of the cell aggregation toward becoming multi-layered, which information is outputted from the output unit 130 and displayed on the display panel 72 or the like.

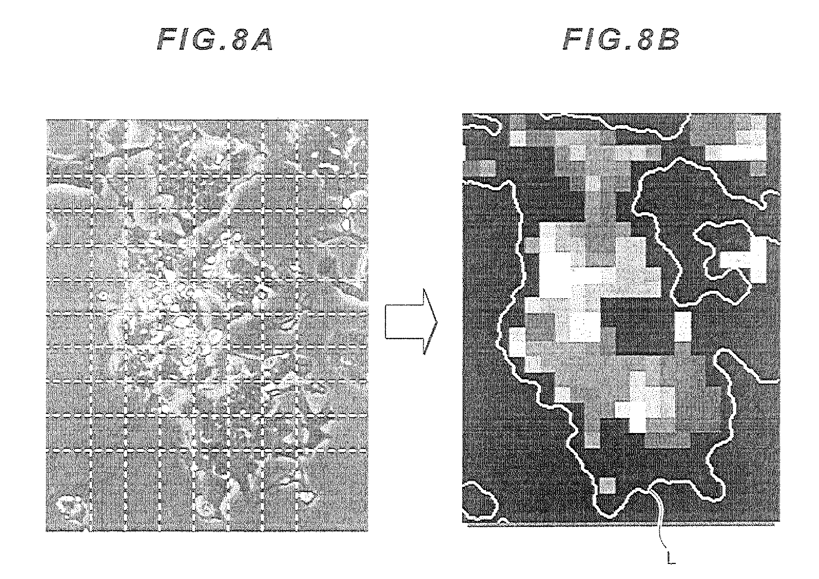

The dotted line in FIG. 8A illustrates an example of the size of the local region, and FIG. 8B illustrates an example of the output of the multi-layering information. This example of the output of the multi-layering information is displayed as the outer contour line L of the cell aggregation of the second image and also as a multi-level gradation display in accordance with the correlation value of the representative degree of approximation, where a site having a high correlation value is dark and a site having a low correlation value is bright. A similar multi-level gradation display is also possible in a case where the difference or other parameter is used as the degree of approximation.

From a display image of the multi-layering information of such description, higher luminosity in the interior of a cell aggregation surrounded by the outer contour line (a shade closer to white on the grey scale) equates to a further degree to which the multi-layering can be decided to have progressed. It is possible for a decision to be made from a visual input in regard to the degree to which the multi-layering has progressed on a given site of the cell aggregation.

Other examples of the output of the multi-layering information are illustrated by a mode in which a region where the correlation value of the representative value of approximation is at or below a predetermined threshold is decided to be multi-layered, and the site at which multi-layering has occurred in the second image illustrated in FIG. 5B is identified and displayed enclosed by a white-bordered box; by a mode in which color coding or the like is used to identify and display multi-layered regions and single-layered regions; or by other modes.

The configuration may be such that the spatial change (disparity) of luminance is calculated for the region of the position used by block matching as the representative degree of approximation, from the luminance distribution of the region of such position; when the correlation value of the representative degree of approximation is at or below a predetermined threshold and at least the spatial change in luminance in the second image is at or above a predetermined threshold, the region of the relevant position is decided to have become multi-layered, and in the identification display the multi-layered region is surrounded by a wh3.te-bordered box or the like in the second image. Examples of the criteria for the spatial change in luminance include a variance of pixel values and/or a derivative sum of the pixel values relative to the spatial direction. This makes use of the fact that a site in a single-layered state has a small spatial change in luminance whereas a site that has become multi-layered has a greater spatial change in luminance.

According to such a configuration, the local region having already become multi-layered in the first image, it is possible to make a more accurate identification and decision, for a site such as in which the representative degree of approximation as calculated by block matching reaches a moderate correlation value (an intermediate color in the grey scale), as to whether or not the relevant site has become multi-layered. A region of a position at which the spatial change in luminance is appreciable is a site where multi-layering has occurred, or otherwise a portion of boundary between the interior and exterior of the cell aggregation; however, the boundary portion reaches a high correlation value of the representative degree of approximation as calculated by block matching (approaches 1), and therefore is omitted in the above-described identification decision of the state of change toward becoming multi-layered.

Thus, examples of multi-layering information for a case in which a multi-layered region is determined and displayed include position information of the multi-layered region in the cell aggregation (for example, the X-Y coordinate position), or information on the size of the cell aggregation that accounts for the multi-layered region (the surface area, the volume, the ratio thereof, or other parameters). Having such numerical data outputted and displayed on the display panel 72 or the like is also a preferred mode in regard to deciding the multi-layered state in a quantitative manner.

The above is an illustration of an instance of analysis in the state in which a specific cell aggregation is selected from the observation image, enlarged, and displayed; however, in a case such as where the image includes a plurality of cell aggregations, such as in FIG. 6, and where there is no particular designation of the analysis range, a similar multi-layering analysis is executed for each of the cell aggregations included in the Observation image. In such a case, the display screen is switched, whereby it is possible to display the state of change of each of the individual cell aggregations toward becoming multi-layered, or it is possible to identify, in the displayed observation image, between a cell aggregation that includes a site where multi-layering has occurred and a cell aggregation that does not include one.

Examples of display modes include display modes in which, for example, for distinction, a cell aggregation having a site where multi-layering has occurred is displayed as being yellow and a cell aggregation without any sites where multi-layering has occurred is displayed as blue, or in which the identification is displayed in accordance with the ratio of the surface area of each cell aggregation that accounts for a site where multi-layering has occurred, where a cell aggregation with a higher surface area ratio is redder, and progresses from yellow to green to blue as the surface area ratio decreases. The configuration may be such that the analysis results are outputted to and recorded using a printer or the RAM 63, a magnetic recording medium, or the like; or outputted outside the system via the communication unit 65.

The observer is thereby able to make a quantitative, visual decision as to the state of change of a cell aggregation toward becoming multi-layered, as included in the image. Thus, in the image processing device 100, block matching that uses the luminance distribution of the local region of the first image as a standard is performed for the first image and the second image in which images of the cell aggregation are taken by an imaging device at a predetermined time interval, the state of change of the cell aggregation toward becoming multi-layered being determined on the basis of the greatest representative degree of approximation. Therefore, according to the technique for determining the state of a cell aggregation using the image processing device 100, there can be provided means by which the state of change of a cell aggregation toward becoming multi-layered can be determined from a small number of images taken by an imaging device without the cells being damaged due to the administration of a reagent.

The embodiment described above provides an example of a configuration of the cell culture observation system BS in which time lapse images (image data) that have been taken with an imaging device and stored in the RAM 63 are read out and the state of change toward becoming multi-layered is analyzed. However, the configuration may be such that images taken by an imaging device are sequentially analyzed in real time as the first and second images, or the configuration may be such that images that have been taken in another observation system and recorded in a magnetic storage medium or the like are read out and the state of change toward becoming multi-layered is analyzed. The configuration may also be such that an operator uses a mouse or the like to set a predetermined range of the first image (for example, a specific cell aggregation, or a specific site in a cell aggregation) as an analysis range, and the image processing device executes an analysis of the state of change toward becoming multi-layered for the analysis range that has been set.

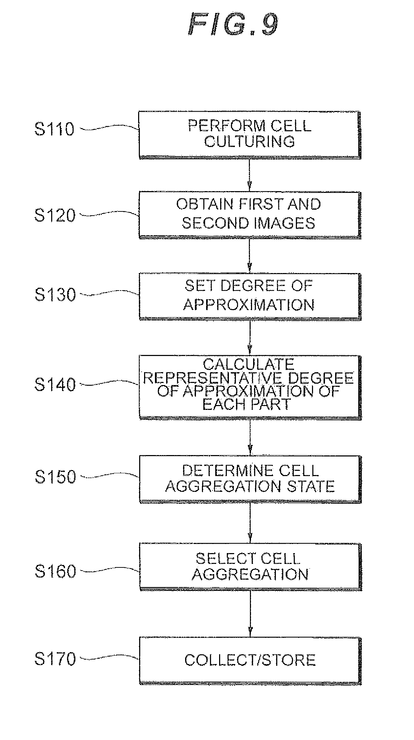

The following is a description of the method for producing a cell aggregation according to an embodiment of the present invention, with reference to FIG. 9. Specifically, the method for producing a cell aggregation comprises a cell culture step for culturing cells (S110) and a determination step for observing, using the above-described image processing device, the cells cultured in the cell culture step and determining the state of change of a cell aggregation toward becoming multi-layered in the cells, which vary by cell culture (S120-S140).

More specifically, the method for producing a cell aggregation is configured to comprise a cell culture step for culturing cells (S110), an obtainment step for obtaining- a first image and a second image taken by photographing, at a predetermined time interval by an imaging device, the cells cultured in the cell culture step (S120); a degree of approximation setting step for performing block matching, using the luminance distribution of a local region in the cell aggregation of the first image as a standard, of the luminance distribution in a nearby part including a position corresponding to the local region in the cell aggregation of the second image, where the degree of approximation of the region exhibiting the highest degree of matching serves as a representative degree of approximation of the relevant local region (S130) ; a calculation step for moving the local region in the first image and calculating the representative degree of approximation of each of the parts of the cell aggregation (S140); a determination step for determining the state of change of a cell aggregation toward becoming multi-layered in accordance with the representative degree of approximation of each of the parts of the cell aggregation as calculated in the calculation step (S150); a selection step for selecting a cell aggregation on the basis of a predetermined standard (S160); and a collection and storage step for collecting and storing the selected cell aggregation (S170). The cells that are cultured may be human-derived cells; cells derived from cows, horses, pigs, mice, or other animals; or plant-derived cells. The cell aggregation may be stored using cryogenic storage.

### EXPLANATION OF NUMERALS AND CHARACTERS

- A:: Local region
- BS:: Cell culture observation system
- GP:: Image processing program
- MC:: Cell aggregation
- 5:: Observation unit
- 6:: Control unit
- 54:: Macro observation system
- 54c:: Imaging device
- 55:: Microscope observation system
- 55c:: Imaging device
- 61:: CPU (computer)
- 62:: ROM
- 63:: RAM
- 100:: Image processing device
- 120:: Image analysis unit
- 130:: Output unit

## Claims

1. A technique for determining the state of a cell aggregation, comprising the steps of:
obtaining a first image and a second image taken by photographing a cell aggregation at a predetermined time interval by an imaging device;
performing block matching, using the luminance distribution of a local region in the cell aggregation of the first image as a standard, of the luminance distribution in a nearby part including a corresponding position in the cell aggregation of the second image, the degree of approximation of the region exhibiting the highest degree of matching serving as a representative degree of approximation of the region of the relevant position;
moving the local region in the first image and calculating the representative degree of approximation of individual parts of the cell aggregation; and
deciding a state of change of the cell aggregation toward becoming multi-layered in accordance with the calculated representative degree of approximation of each of the parts of the cell aggregation.

2. The technique for determining the state of a cell aggregation according to Claim 1, **characterized in that**:
the degree of approximation is a correlation value, and in a case where the correlation value of the representative degree of approximation is at or below a threshold, multi-layering is decided to have occurred in the relevant site.

3. The technique for determining the state of a cell aggregation according to Claim 1, **characterized in that**:
the degree of approximation is a difference, and in a case where the difference of the representative degree of approximation is at or above a threshold, multi-layering is decided to have occurred in the relevant site.

4. An image processing program that can be read out by a computer, the image processing program being adapted for causing the computer to function as an image processing device for obtaining an image taken by an imaging device and performing image processing, comprising the steps of:
obtaining a first image and a second image taken by photographing a cell aggregation at a predetermined time interval by an Imaging device;
performing block matching, using the luminance distribution of a local region in the cell aggregation of the first image as a standard, of the luminance distribution in a vicinity including a corresponding position in the cell aggregation of the second image, having the degree of approximation of the region exhibiting the highest degree of matching serve as a representative degree of approximation of the region of the relevant position, moving the local region in the first image, and calculating the representative degree of approximation of individual parts of the cell aggregation; and
outputting multi-layering information in accordance with the calculated representative degree of approximation;
the image processing program being adapted for causing the computer to function so as to output the multi-layering information by which the state of change of a cell aggregation toward becoming multi-layered can be decided from the obtained first image and the second image.

5. The image processing program according to Claim 4, **characterized in that**:
the degree of approximation is a correlation value; and the step for outputting the ninlti-layering information is configured such that in a case where the correlation value of the representative degree of approximation is at or below a threshold, multi-layering information whereby multi-layering is decided to have occurred in the relevant site is outputted.

6. The image processing program according to Claim 4, **characterized in that:**
the degree of approximation is a difference; and the step for outputting the multi-layering information is configured such that in a case where the difference of the representative degree of approximation is at or above a threshold, multi-layering information whereby multi-layering is decided to have occurred in the relevant site is outputted.

7. The image processing program according to Claim 5 or 6, **characterized in that**:
the step for outputting the multi-layering information is configured such that information on the position in the cell aggregation of the site where mlti-layering is decided to have occurred is outputted.

8. The image processing program according to any of Claims 5 to 7, **characterized in that**:
the step for outputting the multi-layering information is configured such that information is outputted in regard to the size of the cell aggregation that accounts for the site where multi-layering is decided to have occurred.

9. The image processing program according to any of Claims 5 to 8, **characterized in that** in a case where the first image and the second image include a plurality of cell aggregations,
the step for outputting the multi-layering information is configured such that the states of change of each of the cell aggregations toward becoming multi-layered are determined and a distinction is made between cell aggregations having a multi-layered site and cell aggregations not having a multi-layered site, and the results of the distinction are outputted.

10. An image processing device provided with an image analysis unit for obtaining a first image and a second image taken by photographing a cell aggregation at a predetermined time interval by an imaging device, and analyzing the images, and an output unit for outputting the analysis results from the image analysis unit,
the image processing device being configured such that:
the image analysis unit performs block matching, using the luminance distribution of a local region in the cell aggregation of the first image as a standard, of the luminance distribution in a vicinity including a corresponding position in the cell aggregation of the second image, has the degree of approximation of the region exhibiting the highest degree of matching serve as a representative degree of approximation of the region of the relevant position, moves the local region in the first image, calculates the representative degree of approximation of individual parts of the cell aggregation, and generates multi-layering information in accordance with the calculated representative degree of approximation of each of the parts of the cell aggregation; and
the output unit outputs multi-layering information of the cell aggregation generated by the image analysis unit.

11. The image processing device according to Claim 10, **characterized in that**:
the degree of approximation is a correlation value; and the image analysis unit is configured such that, in a case where the correlation value of the representative degree of approximation is at or below a threshold, multi-layering information whereby multi-layering is decided to have occurred in the relevant site is generated.

12. The image processing device according to Claim 10, **characterized in** being configured such that:
the degree of approximation is a difference; and the image analysis unit is configured such that, in a case where the difference of the representative degree of approximation is at or above a threshold, multi-layering information whereby multi-layering is decided to have occurred in the relevant site is outputted.

13. The image processing device according to Claim 11 or 12, **characterized in that**:
the image analysis unit is configured so as to calculate the position in a cell aggregation of the site where multi-layering is decided to have occurred; and
the output unit is configured so as to output information on the position of multi-layering as calculated by the image analysis unit.

14. The image processing device according to any of Claims 11 to 13, **characterized in that**:
the image analysis unit is configured so as to calculate information on the size in the cell aggregation that accounts for the site where multi-layering is decided to have occurred; and
the output unit is configured so as to output the information on the size of multi-layering as calculated by the image analysis unit.

15. The image processing device according to any of Claims 11 to 14, **characterized in that**:
in a case where the first image and the second image include a plurality of cell aggregations,
the image analysis unit is configured so as to determine the states of change of each of the cell aggregations toward becoming multi-layered and distinguish between cell aggregations having a multi-layered site and cell aggregations not having a multi-layered site; and
the output unit is configured so as to output the results of the distinguishing.

16. A method for producing a cell aggregation, comprising:
a cell culture step for culturing cells; and
a determination step for observing, by using the image processing device according to any of Claims 10 to 15, the cells cultured in the cell culture step, and for determining the state of change of a cell aggregation toward becoming multi-layered in the cells, which vary by cell culture.

17. A method for producing a cell aggregation, comprising:
a cell culture step for culturing cells;
an obtainment step for obtaining a first image and a second image taken by photographing, at a predetermined time interval by an imaging device, the cells cultured in the cell culture step;
a degree of approximation setting step for performing block matching, using the luminance distribution of a local region in the cell aggregation of the first image as a standard, of the luminance distribution in a nearby part including a position corresponding to the local region in the cell aggregation of the second image, and having the degree of approximation of the region exhibiting the highest degree of matching serve as a representative degree of approximation of the relevant local region;
a calculation step for moving the local region in the first image, and calculating the representative degree of approximation of individual parts of the cell aggregation; and
a determination step for determining the state of change of a cell aggregation toward becoming multi-layered in accordance with the representative degree of approximation of each of the parts of the cell aggregation as calculated in the calculation step.
